Europäisches Patentamt

European Patent Office

Office européen des brevets

(11) Veröffentlichungsnummer: **0 211 241**
**A2**

## (12) EUROPÄISCHE PATENTANMELDUNG

(21) Anmeldenummer: 86109087.6

(22) Anmeldetag: 03.07.86

(51) Int.Cl.⁴: **C 12 N 9/00**
C 12 N 9/28, C 12 N 9/54
C 12 N 1/24

(30) Priorität: 06.07.85 DE 3524273
19.04.86 DE 3613311

(43) Veröffentlichungstag der Anmeldung:
25.02.87 Patentblatt 87/9

(84) Benannte Vertragsstaaten:
AT CH DE FR GB IT LI NL

(71) Anmelder: Kernforschungsanlage Jülich Gesellschaft
mit beschränkter Haftung
Postfach 1913
D-5170 Jülich 1(DE)

(72) Erfinder: Memmert, Klaus
Artilleriestrasse 40
D-5170 Jülich(DE)

(72) Erfinder: Wandrey, Christian, Prof.
Wolfshovener Strasse 139
D-5170 Jülich(DE)

(54) Verfahren zur Exoenzymgewinnung durch Bakterienkultur.

(57) Die kontinuierliche Gewinnung von Exoenzymen, wie Proteasen, Xylanasen, Amylasen etc. durch Kultivierung von exoenzymproduzierenden Bakterien erfolgt einstufig in einem mit kontinuierlichem Durchfluß betriebenen Fermenter, in dem ein maximaler Enzymproduktivität entsprechender Mangelzustand herbeigeführt wird. Dieser kann intermittierend durch periodische bzw. wiederkehrende Konzentrationsänderungen der Biomasse um $\geq$ 30 % durchlaufen werden, die durch eine periodische Aufprägung eines wachstumsinhibierenden Einflusses, bes. einfach durch eine entsprechende pH-Änderung, jeweils für eine begrenzte Zeitdauer von etwa 0,01 - 5, insbesondere 0,1 - 1 Verweilzeiten bei einer Periodendauer von etwa 1 - 10, insbesondere 6 - 8 Verweilzeiten erreicht werden. Zur Steuerung kan sowohl die optische Dichte der Kultur (als Maß für die Biomassedichte) als auch die Exoenzymkonzentration herangezogen werden. Besonders zweckmäßig wird jedoch sauerstoff-limitiert gearbeitet und der Mangelzustand kontinuierlich durch Einflußnahme auf den Sauerstoff-Eintrag aufrechterhalten, insbesondere durch Steuerung desselben über die dem produktiven Zustand entsprechende Redox-Spannung der Kultur.

./...

FIG. 1

Verfahren zur Exoenzymgewinnung
durch Bakterienkultur

---

Die Erfindung bezieht sich auf Verfahren
zur kontinuierlichen Gewinnung von Exoenzymen
durch Kultivierung von exoenzymproduzierenden
Bakterien in einem Fermenter mit kontinuierlichem Durchfluß.

Exoenzyme wie Proteasen, Xylanasen, Amylasen
etc. werden durch Kultivierung von Bakterien,
insbesondere von Stämmen der Gattung Bacillus
gewonnen. Ihre Herstellung erfolgt bis
heute im allgemeinen lediglich in diskontinuierlicher (Batch-)Fermentation, da die
Ausschüttung dieser Enzyme durch die Organismen in kontinuierlicher Kultur (Chemostat)
rasch nachläßt (F.G. Priest, Bacteriological
Reviews, Bd. 41, No. 3, S. 711 - 753).

Die Gründe für das Nachlassen der Exoenzymausschüttung, sobald man eine Batchfermentation in eine kontinuierliche Fermentation
überführt, sind noch nicht vollständig
geklärt. Die Beobachtung, daß die Exoenzymproduktion in der Batchkultur hauptsächlich
in der spätlogarithmischen und stationären
Phase sowie vor der Sporenbildung stattfindet, nicht dagegen in der logarithmischen Wachstumsphase (B.N. Dancer u. J. Mandelstam, J. Bacteriol. 121 (1975) Seiten 411 - 415), läßt darauf schließen, daß Exoenzyme im allgemeinen zwar konstitutiv gebildet werden, jedoch einer Katabolitrepression unterliegen (P. Schaeffer, Bacteriol. Rev. 33 (1969) Seiten 48 - 71). Für eine Katabolitrepression spricht auch die Beobachtung, daß die Exoenzymproduktion desto höher ist, je schwerer die C-Quelle im Medium für die Organismen zu verwerten ist (V. Moses und P.B. Sharp, J. Gen. Microbiol. 71 (1972) Seiten 181 - 190) d.h., die Verwendung von Glycerin oder Lactose als C-Quelle führt zu einer besseren Enzymausbeute als die Verwendung von Glucose.

Ausgehend von einer solchen Katabolitrepression wurden verschiedentlich mehrstufige kontinuierliche Fermentationsverfahren vorgeschlagen (J.A. Tsaplina u. L.G. Loginova, in: 5. Int. Fermentation Symp. Versuchs- und Lehranstalt für Spiritusfabrikation und Fermentationstechnologie, Berlin, Herausg.: Dellweg, Seite 262), die jedoch für eine technische Produktion nicht befriedigen.

Der Erfindung liegt daher die Aufgabe zugrunde, ein technisch brauchbares kontinuierliches Verfahren vorzusehen.

Diese Aufgabe wird erfindungsgemäß dadurch gelöst, daß man die Fermentation einstufig durchführt und dabei entweder a) fortlaufend für eine maximaler Exoenzymproduktivität entsprechende Wachstumslimitierung sorgt oder b) durch Inhibierung des Wachstums wiederkehrende bzw. periodische Konzentrationsänderungen der Biomasse um $\geq$ 30 % mit jeweiligem Wiederanstieg der Konzentration herbeiführt.

Es hat sich überraschenderweise gezeigt, daß die bei herkömmlicher Arbeitsweise mit Erreichen des steady-states der Fermentation schon weitgehend abgeklungene Exoenzymproduktion aufrechterhalten bzw. wieder angeregt werden kann, wenn die Kultur einem bestimmten Mangelzustand ausgesetzt wird.

Ein solcher Zustand kann, wie weiter unten näher ausgeführt wird,
(1) durch eine auf Übergangszustände der Kultur empfindlich ansprechende Steuerung kontinuierlich eingeregelt oder
(2) intermittierend erreicht werden, indem man in der Kultur wiederkehrend einen auf eine Dezimierung folgenden Vermehrungszustand mit Konzentrationsänderungen der Biomasse um $\geq$ 30 % herbeiführt.

Ein solcher wiederkehrender, insbesondere periodischer Wechsel von Dezimierungs- und Vermehrungsphasen stellt sich in der Kultur selbsttätig ein, wenn man zumindest eine der notwendigen Wachstumsbedingungen bis zu einem solchen Punkt drosselt, an dem Biomassepulsationen bzw. periodische Zu- und Abnahmen der Biomasse in Form sogenannter "Eigenschwingungen" auftreten.

Besonders zweckmäßig wird die wiederkehrende Dezimierung jedoch durch eine wiederkehrende mehr oder minder kurzzeitige Aufprägung eines wachstumsinhibierenden Einflusses hervorgerufen, wie z.B. durch Auslenkung des pH-Wertes oder der Temperatur aus dem Toleranzbereich oder durch Drosselung notwendiger Nährstoffe und dergleichen. Die Einwirkdauer des wachstumsinhibierenden Einflusses soll dabei zur Herbeiführung der gewollten Biomasseminderung ausreichen, die auf den inhibierenden Einfluß hin - ggf. zeitlich verzögert - erreicht wird.

Zeitliches Programm und anzuwendende Bedingungen hängen von den Kulturbedingungen ab und insbesondere von den Durchflußraten, bzw. Verweilzeiten: Besonders zweckmäßig ist eine Periodendauer von 1 bis 10, insbesondere 6 bis 8 Verweilzeiten und eine Einwirkdauer des wachstumsinhibierenden Einflusses von 0,01 bis 5 insbesondere 0, 1 bis 1 und speziell für etwa 0,5 Verweilzeiten, wobei Durchflußraten im Bereich von 0,01 bis 2 $h^{-1}$, insbesondere 0,05 bis 1 $h^{-1}$ und speziell 0,1 bis 0,5 $h^{-1}$ zweckmäßig sind.

Für eine kontinuierliche Produktion kann einfach die der Biomassedichte entsprechende optische Dichte der Kultur kontinuierlich überwacht und zur Steuerung herangezogen werden: Auf diese Weise wird die Annäherung an den steady-state-Wert (Biomassedichteänderung pro Verweilzeit < 20 %) frühzeitig erkannt und jeweils zur Auslösung des wachstumsinhibierenden Einflusses genutzt. Es erfolgt dann - aufgrund der "Streßbedingung" - jeweils ein starker Abfall der Biomassedichte noch über den Zeitraum der Streßphase hinaus, mit nachfolgender Erholung und erneuter steiler Zunahme der Eyoenzymproduktion. Zur Feinsteuerung kann zusätzlich die Exoenzymkonzentration überwacht werden, wobei ein Abfall der Exoenzymkonzentration auf weniger als etwa 1/2 des vorangehenden Maximalwertes als Signal für die Auslösung des wachstumsinhibierenden Einflusses benutzt wird, der aufrechterhalten wird, bis die Biomassedichte auf weniger als etwa 2/3 ihres steady-state-Wertes abgefallen ist.

Nähere Untersuchungen der Einzelperiode haben nun überraschend ergeben, daß das Enzym erst produziert wird, wenn eine bestimmte Sauerstoff-Limitierung einsetzt, die allerdings erst bei empfindlicher Messung über die Redoxspannung erkennbar wird und daß eine enge Beziehung zwischen der Enzymproduktivität und der Sauerstoff-Limitierung besteht.

Davon ausgehend kann eine praktisch stetige Enzymproduktion erreicht werden, wenn man in der Kultur fortlaufend eine maximaler Exoenzymproduktion entsprechende Sauerstoff-Limitierung aufrechterhält.

Zu diesem Zweck wird insbesondere die den Sauerstoffgehalt der Kultur sehr empfindlich anzeigende Redoxspannung im Fermenter überwacht und als Regelparameter für den Sauerstoffeintrag benutzt, der insbesondere über die Rührgeschwindigkeit variiert wird. Zweckmäßig sind Rührgeschwindigkeiten von 0 bis $\sim$ 1000 Upm bei einer Belüftung mit 0,3 bis 3 VVm, insbesondere 1 bis 2 VVm.

Nützlicherweise enthält die Kultur ein Antischaummittel bekannter Art, wie z.B. Polypropylenglycol oder Siliconöl, um ggf. stärkere Schaumbildung bei dem über die Rührgeschwindigkeit geregelten Sauerstoffeintrag zu unterdrücken.

Die maximaler Exoenzymproduktion entsprechende Redoxspannung (bzw. das Redoxpotential) hängt - dem Betrag nach - selbstverständlich von den für die Fermentation gewählten Parametern, wie insbesondere pH-Wert, Nährstoffzusammensetzung und -konzentration, Belüftungsrate, speziellem Mikroorganismus, Temperatur etc. ab.

D.h., der jeweils einzuregelnde Sauerstoffeintrag (bzw. die jeweils einzuhaltende Redoxspannung) wird für eine spezielle Produktion tunlichst zu Beginn unmittelbar im jeweiligen Fermentationssystem bestimmt.

Da durch das Prinzip der Redox-Steuerung des Sauerstoffeintrags verhindert wird, daß die Bakterienkultur in einen steady-state einläuft, die Kultur also die Tendenz zeigt, in ihrer Konzentration immer weiter zuzunehmen oder aber auszuwaschen, ist es bei dieser Verfahrensweise sehr nützlich, die Biomassekonzentration durch geeignete Maßnahmen im gewünschten Rahmen zu halten.

Zu diesem Zweck wird insbesondere eine zweite Regelung vorgesehen, bei der die Biomassekonzentration im Fermenter kontinuierlich (z.B. über die optische Dichte) überwacht und durch Variation der Verweilzeit im Fermenter und/oder der Sauerstofflimitierung über die Redoxspannung der Kultur geregelt wird. Die Redoxspannung soll dabei selbstverständlich im Bereich optimaler Enzymproduktion bleiben.

Vorzugsweise wird bei der erfindungsgemäßen sauerstofflimitierten Exoenzymbildung im Durchflußfermenter für eine möglichst hohe Biomassekonzentration im Reaktor gesorgt, die jedoch noch keiner Nährstofflimitierung (Kohlenstoff, Stickstoff etc.) unterliegt. Zusätzlich kann die Biomasse nach bekannten Verfahren zurückgehalten und über eine Variation der Rückhalterate eine optimale Biomassekonzentration aufrechterhalten werden.

Als Mikroorganismen dienen dabei exoenzymproduzierende Bakterien, insbesondere der Gattung Bacillus. Speziell untersucht wurde die Art Bacillus amyloliquefaciens, Stämme DSM 7 und DSM 1061.
Die Temperatur liegt insbesondere im Bereich von 30 bis 37°C.

Weitere Besonderheiten der Erfindung gehen aus der nachfolgenden Beschreibung anhand von Beispielen unter Bezugnahme auf die angefügten Zeichnungen hervor; diese zeigen Kurvenbilder für den zeitlichen Verlauf von:

Figur 1    einer kontinuierlichen Xylanasebildung gemäß der Erfindung;

Figur 2    einer ungesteuerten Xylanasebildung
           herkömmlicher Art in einem Durchflußfermenter;

Figur 3    optischer Dichte, Xylanasekonzentration und Glycerinkonzentration
           bei kontinuierlicher Fermentation
           mit selbsttätiger Biomassepulsation;

Figur 4    optischer Dichte, Exoenzymkonzentration und pH-Wert bei kontinuier-
           licher Fermentation mit wiederkeh-
           render kurzzeitiger Wachstumsinhi-
           bierung für Xylanase, Amylase
           und Protease;

Figur 5    optischer Dichte, Xylanasekonzentration, Glycerinkonzentration und
           pH-Wert bei kontinuierlicher Fermen-
           tation mit wiederkehrender kurzzei-
           tiger Wachstumsinhibierung; und

Figur 6    Kurven für die quasikontinuierliche
           Xylanasebildlung durch wiederkeh-
           rende pH-Absenkung mit gleichzeiti-
           gem Redoxspannungsauftrag.

In den nachfolgend beschriebenen Beispielen wurden jeweils Bakterien vom Stamm Bacillus amyloliquefaciens DSM 7 in einem Rührkessel-reaktor (Fermenter) unter folgenden Bedin-gungen kultiviert:

7 1-Fermenter      (Typ BIOSTAT"E" der
                   Fa. B. BRAUN, Melsungen)
Arbeitsvolumen     4 1 (Beisp. 1-3) bzw.
                   3 1 (Beisp. 4-5)
Temperatur         37°C
Belüftung          6 1/min Druckluft
                   [1,5 VVm (Beisp. 1-3)
                   bzw. 2 VVm (Beisp.
                   4-5)]
Rührertyp          Turbo-Blattrührer
Drehzahl           0 Upm bei Beisp.
                   1-3
                   (ausreichende Durchmi-
                   schung durch Zuluft)
                   ab Beisp. 4: 300 Upm,
                   wenn nicht vom Regler
                   angesteuert.

Das zuvor sterilisierte Medium hatte folgende
Zusammensetzung:

| 20 | g | Glycerin |
|---|---|---|
| 6 | g | $(NH_4)_2SO_4$ |
| 1,67 | g | $NaNO_3$ |
| 0,52 | g | $Mg(NO_3)_2 \cdot 7\,H_2O$ |
| 1 | g | Hefeextrakt |
| 2,72 | g | $KH_2PO_4$ |
| 0,5 | g | Zitronensäure |
| 72 | mg | $CaCl_2 \cdot 2H_2O$ |
| 10 | mg | $FeSO_4 \cdot 7\,H_2O$ |
| 5 | mg | $MnSO_4 \cdot H_2O$ |

$H_2O$   ad   1000 ml

Zu Beispiel 4 und 5 (mit Rührerbetrieb)
waren zusätzlich in 1 Liter 0,083 ml Polypropylenglycol 2000
0,083 ml Silicon-Antischaum-Emulsion M 30
        (Fa. Serva)

Vergleichsbeispiel

Der Fermenter wurde zum Zeitpunkt t = 0 h
mit 200 ml einer Bacillus-Vorkultur, die
in einem Schüttelkolben herangezogen worden
war, beimpft und zunächst für 16 Stunden
als Batchkultur (diskontinuierlich) betrieben.

Ab t = 16 h wurde die Fermentation kontinuierlich durchgeführt, wobei ein konstanter
Volumenstrom frischen Mediums in den Fermenter gepumpt und die gleiche Menge Fermenterinhalt kontinuierlich abgelassen wurde.
Der konstante Volumenstrom betrug 220 ml/h
($D = 0,055 \ h^{-1}$, $\tau = 18,2 \ h$). Der pH-Wert
wurde auf pH 6,0 konstant geregelt.

Diese Bedingungen wurden für 13 Verweilzeiten
konstant gehalten.

Dabei zeigte sich bei etwa gleichbleibender
Biomassekonzentration (steady-state) ein
Absinken der Xylanasekonzentration von
11 U/ml auf Werte unter 0,5 U/ml (siehe
Fig. 2).

Beispiel 1
Die Fermentation wurde nach Animpfen unter
gleichen Bedingungen, wie im Vergleichsbeispiel beschrieben, zunächst 15 Stunden
lang diskontinuierlich betrieben und danach

0211241

mit 375 ml/h (D = 0,094 h$^{-1}$; $\tau$ = 10,7 h) kontinuierlich betrieben.

Der pH-Wert wurde für die ersten 336 Stunden auf konstant pH 5,5, danach konstant auf pH 5,1 gehalten.

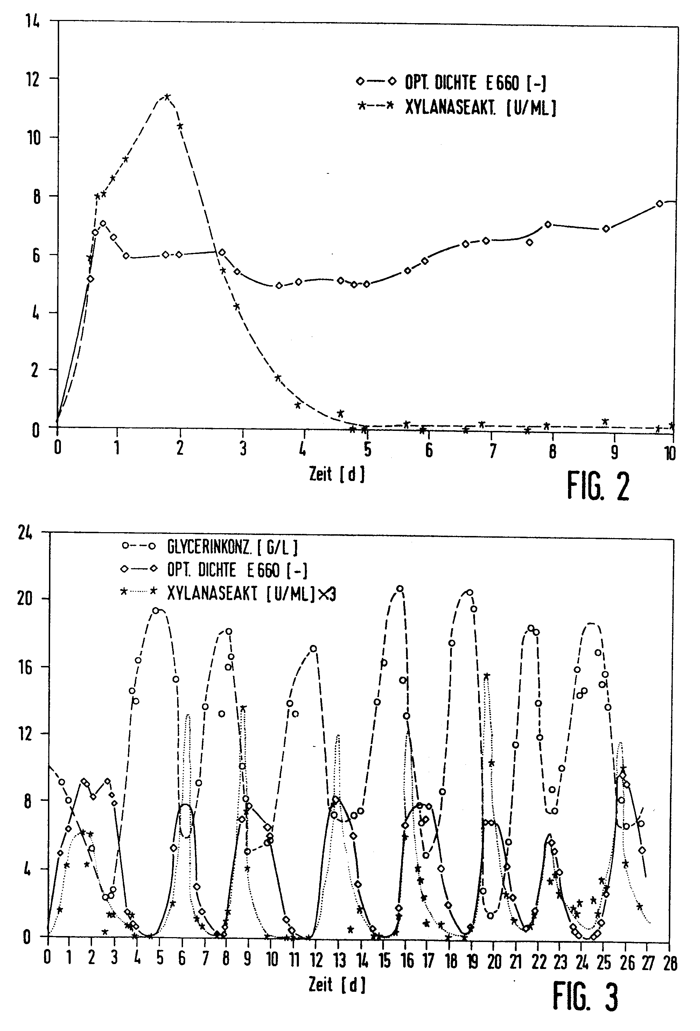

Bei beiden pH-Werten traten im kontinuierlichen Betrieb selbsttätig starke Biomassepulsationen (siehe Fig. 3, opt. Dichte) auf, die besonders durch ein jeweils steiles Wieder-Ansteigen der Biomasse gekennzeichnet waren. Über die gesamte Versuchsdauer war eine wiederkehrende Xylanaseproduktion während des Wiederansteigens der Biomasse zu beobachten. - Im wesentlichen gegenläufig zur optischen Dichte pulsiert die Glycerinkonzentration.

Beispiel 2

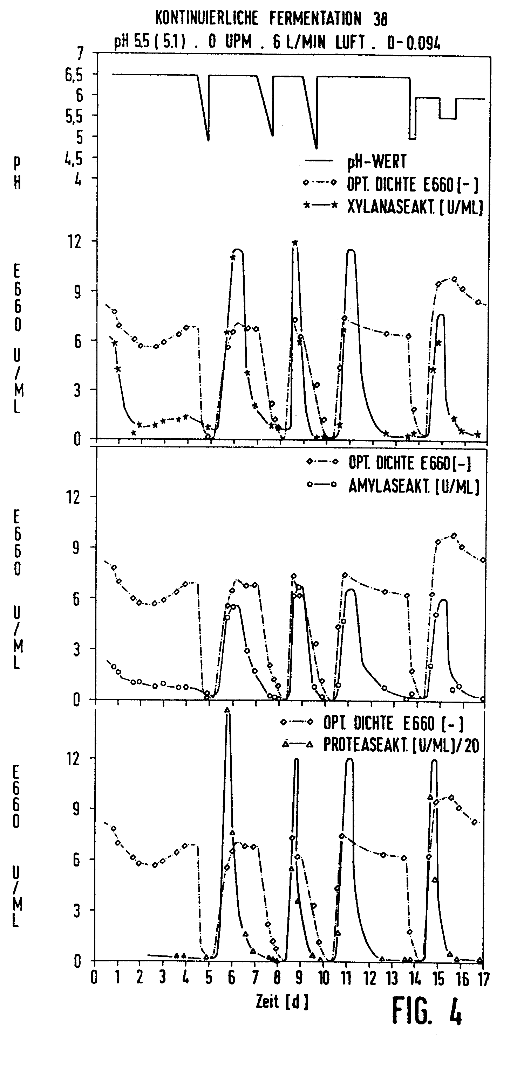

Die Fermentation wurde nach Animpfen unter gleichen Bedingungen, wie im Vergleichsbeispiel beschrieben, von Anfang (t = 0 h) an kontinuierlich mit 400 ml/h (D = 0,1 h$^{-1}$; $\tau$ = 10 h) betrieben. Der pH-Wert wurde zunächst konstant auf pH 6,5 gehalten. Nach Einpendeln der Biomassekonzentration in ein steady-state und nach Absinken der Exoenzymkonzentration wurde die pH-Regelung zum Zeitpunkt t = 107 h für 1 Verweilzeit (10 h) ausgeschaltet. Der pH-Wert sank daraufhin von allein auf pH 5,0. Zum Zeitpunkt t = 117 h wurde die pH-Regelung wieder eingeschaltet und der pH-Wert dadurch sofort wieder auf pH 6,5 korrigiert. Während der pH-Auslenkung aus dem Toleranzbereich (pH 6 bis 8) des Bakteriums war eine starke Abnahme der Biomassekonzentration und danach ein steiles Wiederansteigen der Biomassekonzentration zu beobachten. Letzteres war begleitet von einem deutlichen Ansteigen der Exoenzymkonzentration (siehe Fig. 4 für Xylanase, Protease und Amylase).

Nach erneutem Einpendeln der Biomasse in ein steady-state und wieder nachlassenden Exoenzymkonzentrationen wurde zum Zeitpunkt t = 168 h die pH-Regelung erneut für 1,5 Verweilzeiten (15 h) abgeschaltet.

Der pH-Wert sank von selbst auf pH 5,0 ab und wurde ab t = 183 h durch Wiedereinschalten der pH-Regelung auf konstant pH 6,5 zurückgebracht.

Auch hier wurde wiederum eine Abnahme der Biomassekonzentration, gefolgt von einem steilen Ansteigen der Biomassekonzentration beobachtet, wieder begleitet von einem deutlichen Ansteigen der Exoenzymkonzentrationen.

In entsprechender Weise wurde die pH-Regelung zwischen t = 215 h und t = 229 h für 14 Stunden ausgeschaltet, wodurch der pH-Wert bis auf pH 4,7 absank. Die Biomassekonzentration und die Exoenzymkonzentrationen verhielten sich analog zu den ersten beiden pH-Auslenkungen.

Zum Zeitpunkt t = 325 h wurde der pH-Wert aktiv mittels pH-Regelung durch Säurezugabe auf pH 5,0 abgesenkt und für 0,6 Verweilzeiten (6 Stunden) auf pH 5,0 konstant gehalten. Danach wurde der pH-Wert mittels pH-Reglers auf pH 6,0 angehoben und auf diesem Wert konstant gehalten. Auch bei dieser Verfahrensweise verhielten sich Biomassekonzentration und Exoenzymkonzentrationen wie zuvor bei der selbsttätigen pH-Erniedrigung.

Beispiel 3

Die Fermentation wurde nach Animpfen, wie im Vergleichsbeispiel beschrieben, vom Zeitpunkt t = 0,5 h kontinuierlich mit 370 ml/h (D = 0,093 h$^{-1}$, $\tau$ = 10,8 h) betrieben.

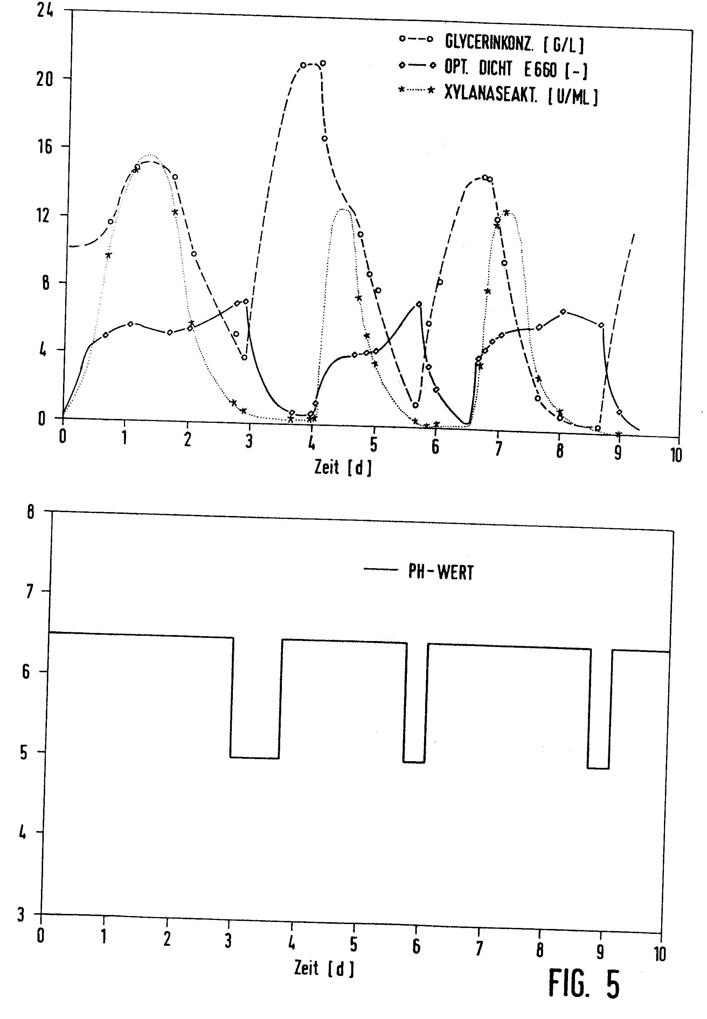

Von t = 69 h an wurde der pH-Wert für 17 Stunden (1,6 Verweilzeiten) und von t = 136 h bzw. t = 208 h an für jeweils 8 Stunden (0,75 Verweilzeiten) mittels pH-Regelung aktiv von konstant pH 6,5 auf konstant pH 5,0 abgesenkt. Das Verhalten von Biomassekonzentration und Xylanasekonzentration entsprach den im Beispiel 2 geschilderten Beobachtungen (siehe Fig. 5).

Weitere Versuche lassen erkennen, daß dieses Verfahren auch bei Verwendung anderer C-Quellen als Glycerin, so z.B. Lactose und Stärke, funktioniert.

Der dem Bacillus amyloliquefaciens artverwandte Bacillus subtilis zeigt ein ähnliches Verhalten.

Obwohl bei den vorstehend beschriebenen Beispielen als wachstumsinhibierender Einfluß zur Biomassedezimierung jeweils eine sehr einfach zu realisierende pH-Erniedrigung gewählt wurde, sind selbstverständlich andere Inhibitionen wie z.B. pH-Erhöhung oder Temperaturauslenkung in gleicher Weise wirksam.

Beispiel 4

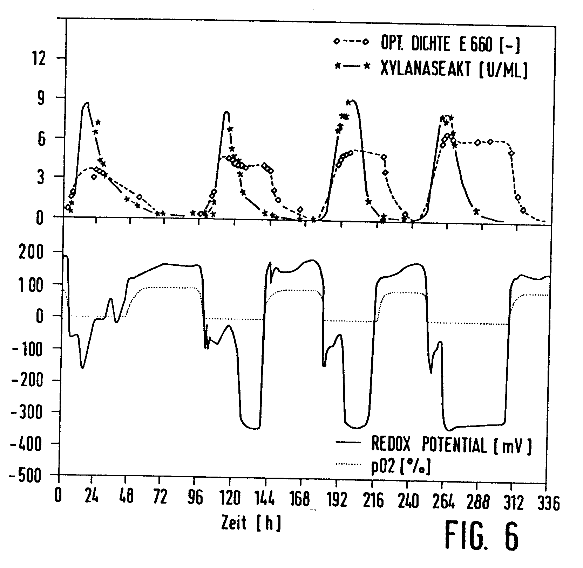

Fig. 6 zeigt den Verlauf der Redoxspannung bei einer, wie vorstehend beschrieben, durch wiederkehrende pH-Absenkung "periodisch angestoßenen" Xylanasebildung im Durchflußfermenter:

Man erkennt, daß maximale Enzymkonzentrationen in der Kultur mit mittleren Redoxspannungen im Abfallbereich übereinstimmen, die bei der vorliegenden Xylanasebildung bei pH 6 und einer Verweilzeit von 5 Stunden in der Gegend von -50 mV lagen.

Beispiel 5

Für die in diesem Beispiel gezeigte Redoxregelung wurde ein PID-Regler (Fa. Foxboro) verwendet, der das Redoxpotential durch Ansteuern der Rührerdrehzahl des Fermenters konstant auf dem vorgegebenen Sollwert hielt.

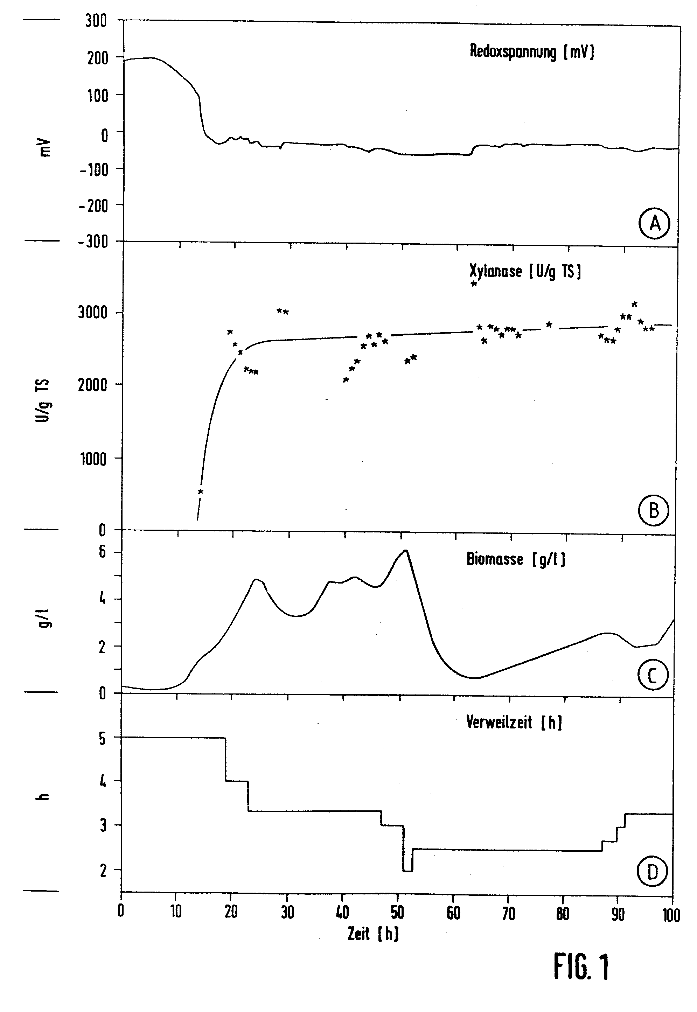

Die Bakterienkultur der über einen längeren Zeitraum kontinuierlich laufenden Fermentation wurde zum Zeitpunkt t = 1960 h bis 1964 h durch eine pH-Absenkung zu einer Biomassedichteabnahme gezwungen und fing ab ca. t = 1977 h wieder an stark zuzunehmen (Fig. 1; Kurve C).

Gleichzeitig mit der Biomassezunahme nahm das Redoxpotential bis ca. t = 1985 h stark ab und und hielt sich danach in einem Bereich von ca. 0 bis -50 mV (Kurve A). Rechtzeitig, bevor das Redoxpotential auf niedrige Werte, wie sie aus Fig. 6 hervorgehen, abstürzen konnte, wurde zum Zeitpunkt t = 1988 h die Redox-Regelung eingeschaltet. Während die Rührerdrehzahl des Fermenters bis dahin konstant bei 300 Upm lag, wurde die Rührerdrehzahl ab t = 1988 h extern vom Redox-Regler angesteuert, um das Redoxpotential auf dem jeweils vorgegebenen Redox-Sollwert zu halten.

Als erster Redox-Sollwert wurde von t = 1988 bis 1992 h -15 mV gewählt. Da dieser Sollwert geringfügig über dem letzten freien Redoxwert lag, konnte die Biomassekonzentration zunächst weiter zunehmen.

Um eine zu starke Biomassezunahme oder -abnahme zu vermeiden, wurde bei t = 1989 h und zu weiteren Zeitpunkten die Verweilzeit der kontinuierlichen Kultur variiert (Kurve D).

Hauptsächlich ebenfalls zur Beeinflussung der Biomassedichte wurde der Redox-Sollwert innerhalb des Bereiches von -15 bis -50 mV variiert.

Während des gesamten Zeitraums der Redoxregelung verlief die Enzymkonzentration etwa
par_allel zur Biomassedichte, so daß die
Enzymausbeute konstant im Bereich von 2000
bis 3000 U Xylanase pro g Biotrockenmasse
lag (Kurve B).

Das vorstehend beschriebene Beispiel bezieht
sich auf die Bildung von Xylanase. Analoge
Verhältnisse wurden für Protease und Amylase
gefunden.

Die Aufarbeitung der Produktlösung des
Fermenters erfolgt nach bekannten Methoden
z.B. durch Zentrifugieren oder Cross-Flow-
Filtration unter kontinuierlicher Abtrennung
der Biomasse von der flüssigen Phase, aus
der dann das Enzym bzw. die Enzyme in bekannter Weise, z.B. durch Aussalzen, abgetrennt und isoliert bzw. gereinigt werden.

Kernforschungsanlage Jülich
Gesellschaft mit beschränkter Haftung

P a t e n t a n s p r ü c h e

1. Verfahren zur kontinuierlichen Gewinnung von Exoenzymen durch Kultivierung von exoenzymproduzierenden Bakterien in einem Fermenter mit kontinuierlichem Durchfluß,
d a d u r c h   g e k e n n z e i c h n e t ,
daß man die Fermentation einstufig durchführt und dabei entweder a) fortlaufend für eine maximaler Exoenzymproduktivität entsprechende Wachstumslimitierung sorgt oder b) durch Inhibierung des Wachstums wiederkehrende bzw. periodische Konzentrationsänderungen der Biomasse um $\gtrsim$ 30 % mit jeweiligem Wiederanstieg der Konzentration herbeiführt.

2. Verfahren nach Anspruch 1,
d a d u r c h   g e k e n n z e i c h n e t ,
daß man in der Kultur fortlaufend eine maximaler Exoenzymproduktion entsprechende Sauerstoff-Limitierung aufrechterhält.

3. Verfahren nach Anspruch 2,
d a d u r c h   g e k e n n z e i c h n e t ,
daß man die maximaler Exoenzymproduktion entsprechenden Sauerstoff-Limitierung durch fortlaufende Regelung des Sauerstoffeintrags abhängig von der kontinuierlich überwachten Redoxspannung der Kultur einhält.

5/
10

ha

4. Verfahren nach einem der Ansprüche 1a, 2 oder 3, d a d u r c h   g e k e n n z e i c h n e t , daß man eine möglichst hohe Biomassekonzentration im Fermenter hält, die jedoch noch keiner Nährstoff-Limitierung unterliegt.

5. Verfahren nach Anspruch 4, d a d u r c h   g e k e n n z e i c h n e t , daß man die Biomassekonzentration mit Hilfe der Verweilzeit im Fermenter und/oder der Redoxspannung der Kultur regelt.

6. Verfahren nach Anspruch 5, d a d u r c h   g e k e n n z e i c h n e t , daß man die Biomassekonzentration zusätzlich durch Biomasserückhaltung unter Variation der Rückhalterate aufrechterhält.

7. Verfahren nach einem der vorangehenden Ansprüche, d a d u r c h   g e k e n n z e i c h n e t , daß man die Redoxspannung bei einem pH-Wert der Kultur von 6,0 zwischen 0 und -100 mV, insbesondere in der Gegend von -15 bis -50 mV, hält.

8. Verfahren nach Anspruch 1, g e k e n n z e i c h n e t   d u r c h eine periodische Aufprägung eines wachstumsinhibierenden Einflusses jeweils für eine derart begrenzte Zeit, daß eine Verminderung der Biomassekonzentration um $\geq$ 30 % erreicht wird.

9. Verfahren nach Anspruch 8, d a d u r c h   g e k e n n z e i c h n e t ,

daß man den wachstumsinhibierenden Einfluß jeweils für eine Zeitdauer von 0,01 bis 5, insbesondere 0,1 bis 1, speziell für etwa 0,5 Verweilzeiten einwirken läßt.

10. Verfahren nach Anspruch 9, g e k e n n z e i c h n e t   d u r c h eine Periodendauer von 1 bis 10, insbesondere 6 bis 8 Verweilzeiten.

11. Verfahren nach einem der Ansprüche 8 bis 10, d a d u r c h   g e k e n n z e i c h n e t , daß man die wachstumsinhibierenden Bedingungen durch eine Auslenkung des pH-Wertes aus dem Toleranzbereich der Bakterien erzeugt.

12. Verfahren nach Anspruch 11, d a d u r c h   g e k e n n z e i c h n e t , daß man den pH-Wert um 0,3 bis 4 pH-Einheiten, insbesondere 0,5 bis 2, speziell um etwa 1 pH-Einheit(en) aus dem Toleranzbereich heraus absenkt.

13. Verfahren nach einem der vorangehenden Ansprüche, d a d u r c h   g e k e n n z e i c h n e t , daß man als Bakterien solche der Art Bacillus amyloliquefaciens oder Bacillus subtilis kultiviert.

14. Verfahren nach Anspruch 13, d a d u r c h   g e k e n n z e i c h n e t , daß man Bacillus amyloliquefaciens DSM 7 oder DSM 1061 kultiviert.

15. Verfahren nach einem der vorangehenden Ansprüche, d a d u r c h   g e k e n n z e i c h n e t ,

4

daß man bei Durchflußraten im Bereich von
0,01 bis 2 h$^{-1}$, insbesondere 0,05 bis 1 h$^{-1}$,
speziell 0,1 bis 0,5 h$^{-1}$ arbeitet.

16. Verfahren nach Anspruch 8,
d a d u r c h   g e k e n n z e i c h n e t ,
daß man die optische Dichte der Kultur (als
Maß für die Biomassedichte) und/oder die Exoenzymkonzentration überwacht und die wachstumsinhibierende Maßnahme ergreift, wenn die Exoenzymkonzentration auf $\leq$ 1/2 des vorangehenden
Maximalwertes sinkt bzw. die Biomassedichteänderung
pro Verweilzeit $\leq$ 20 % liegt, bis die optische
Dichte auf $\leq$ 2/3 abgefallen ist.

17. Verfahren nach Anspruch 13 zur Gewinnung von
Amylase, Xylanase und/oder Protease,
d a d u r c h   g e k e n n z e i c h n e t ,
daß man Bacillus amyloliquefaciens oder subtilis
in einem Rührkesselreaktor im Bereich von 30
bis 37 °C; Belüftung mit 0,3 bis 3 VVm, insbesondere 1 bis 2 VVm; Durchflußraten von 0,05
bis 0,5 h$^{-1}$; pH 6 bis 8; 0 bis 1000 Upm; mit
Lactose, Stärke oder insbesondere Glycerin
als C-Quelle in einem übliche Nährsalze und
Spurenstoffe enthaltenden Nährmedium auf Gleichgewichtsbedingungen anzüchtet und dann entweder
wiederkehrend bei einer Gesamtperiodendauer
von etwa 7 Verweilzeiten einer populationsmindernden Phase von etwa 1 Verweilzeit unterwirft
oder fortlaufend sauerstofflimitiert für maximale
Exoenzymproduktion durch Regelung des Sauerstoffeintrages über die Redoxspannung arbeitet.

FIG. 1

2/5

0211241

FIG. 2

FIG. 3

KONTINUIERLICHE FERMENTATION 38
pH 5,5 (5.1) . 0 UPM . 6 L/MIN LUFT . D-0.094

FIG. 4

FIG. 5

5/5

FIG. 6